# EUROPEAN PATENT SPECIFICATION

(11) **EP 2 637 696 B1**
(45) Date of publication and mention of the grant of the patent: **02.06.2021**
(21) Application number: 11839787.6
(22) Date of filing: 08.11.2011
(51) Int. Cl.: A61K 9/00, A61K 9/20, A61K 9/50, A61K 47/30, A61K 47/58

(54) **SUSTAINED RELEASE COMPOSITIONS**
ZUSAMMENSETZUNGEN MIT VERZÖGERTER FREISETZUNG
COMPOSITION À LIBÉRATION PROLONGÉE

(30) Priority: 10.11.2010 IN 3088MU2010
(43) Date of publication of application: 18.09.2013
(73) Proprietor: Rubicon Research Private Limited, Thane West - 400604 Maharashtra (IN)
(72) Inventor: PILGAONKAR, Pratibha Sudhir, Thane West - 400604 Maharashtra (IN); RUSTOMJEE, Maharukh Tehmasp, Mumbai 400078 (IN); GANDHI, Anilkumar Surendrakumar, Mumbai 400078 (IN)
(74) Representative: Harrison IP Limited
(86) International application number: PCT/IN2011/000764
(87) International publication number: WO 2012/063257

(56) References cited:
- CN-A- 1 931 140
- CN-A- 101 422 611
- CN-A- 101 474 148
- US-A- 4 221 778
- US-A- 5 186 930
- US-A1- 2003 099 711
- US-A1- 2005 181 050
- US-A1- 2009 011 027
- US-A1- 2010 166 858

## Description

### Field of the Invention

The present invention relates to sustained release compositions comprising a sustained release composition comprising a plurality of sustained release beads comprising coated drug-resin complexes comprising a drug-resin complex and a release modifier coating.

### Background of the Invention

The advantages of sustained release formulations are well known in the pharmaceutical field. These include the ability of the given pharmaceutical preparation to maintain a desired therapeutic effect over a comparatively longer period of time, reduced side effects, etc. Moreover, for drugs having a short elimination half-life, less frequent administration and better patient compliance may be obtained with sustained release preparations as compared to the conventional dosage forms.

Dextromethorphan is one of the most widely used antitussives for the treatment of cough associated with acute upper respiratory tract infection. It has an opioid-like structure and being the D-isomer of codeine analog, exerts its pharmacologic action in the same way as L-isomer; but lacks analgesic and addictive properties thereof when used at recommended doses. Dextromethorphan acts centrally to relieve cough, is active against dry cough and does not exhibit significant expectorant properties for productive cough. Dextromethorphan is rapidly absorbed from the gastrointestinal tract and peak plasma concentrations are reached in approximately 2.5 hours. The plasma elimination half-life of dextromethorphan is 1.2 to 3.9 hours. Dextromethorphan is widely distributed, and is rapidly and extensively metabolized by the liver. The maximum time of effectiveness of dextromethorphan in conventional compositions is therefore only a few hours. Consequently, repeated dosages must be taken at frequent intervals to obtain long term therapeutic levels of drug. The usual doses for immediate-release formulations range from 2.5 - 30 mg 3 - 4 times a day. After high initial peak concentrations, the level of drug in the blood stream constantly decreases because of the biological elimination, so that there is little or no therapeutic effect at the end of the period between dosages. As a result, the therapeutic effect fluctuates between dosages resulting in peaks and troughs in the level of drug in the blood. Multiple dosing also often results in poor patient compliance and inefficient therapy

Development of sustained release formulation of active agents such as dextromethorphan that release the drug at a predetermined rate to maintain a desired therapeutic effect over a comparatively longer period of time with reduced side effects thereby increasing patient compliance, minimizing peak-trough fluctuations and improving effectiveness of the medication are therefore particularly desirable. Further sustained release compositions can also reduce night-time coughing, eliminate need to interrupt sleep to take medication and prevent missed doses thereby increasing patient convenience.

Ion-exchange is known to have utility in providing improved drug delivery systems for orally administered drug products. U.S. Patent No. 2,990,332 discloses drug-resin complexes prepared by interaction of cationic ion-exchange resins with basic drugs in their cationic form, such as amphetamine and codeine. Drug-resin complexes thus offered a method for retarding the rate of drug release not available using conventional drug delivery systems. However the degree of control of drug release from drug-resin complexes was found to be unsatisfactory since for many drugs only a relatively short delay in drug release was obtained. Sustained release was difficult to achieve with such uncoated drug-resin complexes due to many variables such as variation in particle size and cross-linkage in the resin used as well as patient to patient variability in the ionic strength and pH of the gastrointestinal fluids that determine the release of the drug from the drug resin complex.

Attempts were therefore made to coat drug-resin complexes so as to provide a further diffusion barrier to drug release and thereby offer greater control over the drug release profile. Various coated drug-ion exchange resin complexes have been reported in U.S. Patent Nos. 3,138,525, 3,499,960 and 3,594,470, but none provide desired sustained release profile as obtainable with the present invention. Moreover such coated drug-resin complexes when suspended in liquid dosage forms or upon contact with gastrointestinal fluids, resulted in rupturing of the diffusion barrier coating due to swelling of the resin leading to loss of control of drug release.

U.S. Patent No. 4,221,778 discloses a range of drug-resin complexes coated with a water-permeable diffusion barrier coating such as ethylcellulose. The use of a solvating agent such as PEG 4000 has also been described therein which minimizes the tendency of drug ion exchange resin complex particles to swell and fracture the barrier coatings in biological fluids. Further, U.S. Patent No. 4,996,047 describes using drug content above a specified value in the drug-ion exchange resin complex to avoid the swelling of the drug-ion exchange resin complex and thereby minimizing the rupture of the coating and avoiding the need to use a solvating agent. U.S. Patent No. 5,368,852 discloses that despite the use of impregnating agents, certain preservatives used in the liquid preparations containing coated drug-resin complexes tend to cause rupture of the diffusion barrier coating of the drug-ion exchange resin complex. Such a rupturing of the coating membrane is disclosed to be avoided first by use of a specific diffusion barrier film material, i.e. ethyl cellulose having a specific content of ethoxyl group, and secondly by adding the specific preservative at a specific concentration to control the release of the drug.

Further various attempts have been made to provide sustained release compositions of active agents comprising either drug-resin complexes coated at a single barrier coating level or combinations thereof with uncoated drug-resin complexes. U.S. Patent No. 6,001,392 discloses sustained release composition comprising a mixture of coated and un-coated drug-resin complexes. About 20 to about 80% of the drug-resin complexes are said to be coated with a mixture of ethyl cellulose or ethyl cellulose latexes with plasticizers and water dispersible polymers. U.S. Publication No. 2008/0118570 discloses a coated drug/resin complex that comprises drug-resin complexes and a substantially plasticizer-free coating layer of a methacrylate polymer preferably Eudragit NE-30D applied at least substantially around a portion of the resin forms to control the release rate of the drug. Suspension dosage forms comprising either completely coated drug-resin complexes coated at a single level or comprising a portion of un-coated and a portion of coated drug-resin complexes have been described herein. Further, U.S. Publication No. 2007/0215511 discloses a coated drug-ion exchange resin matrix useful in preparing modified release formulations, comprising a drug-ion exchange resin complex and a water-insoluble release retardant which forms a drug-resin matrix and a cured water-permeable, high tensile strength, water-insoluble, barrier coating comprising a non-ionic polymer such as Kollicoat SR 30D and a plasticizer. These compositions are said to provide prolonged, programmable release of drugs from the drug-resin complexes of up to about 24 hours. US5186930 discloses sustained release pharmaceutical composition comprising a drug-resin complex suspended in a liquid carrier for oral administration; the drug-resin complex comprises a drug-resin particle coated with a first inner coating of a wax and a second outer coating of a polymer, wherein said drug-resin particle comprises an acidic or basic drug ionically bound to an ion-exchange resin particle. Further, the formulation of US20050181050 discloses drug-resin complex particles comprising an immediate release coating and a delayed release coating. US20030099711, discloses a methylphenidate composition including coated drug-resin complexes.

Since the drug release kinetics from ion exchange resins is dependent on the pH and ionic strength of the fluid of the gastrointestinal tract that varies from patient to patient, the presence of uncoated drug-resin complexes along with coated drug-resin complexes can result in high initial release of the drug from the uncoated drug-resin complexes causing dose dumping and related adverse effects. Additionally, if the sustained release compositions comprise drug-resin complexes that are coated by single level of barrier coating i.e. the entire portion of drug-resin complexes are coated with a barrier diffusion coating to up to for e.g. say 20% or say 30% by weight of the drug-resin complex, then excessive retardation of drug release may be observed and in such instances the percentage of drug released from the coated complexes may be incomplete and substantial amount of the drug may remain bound within the coated drug-resin complexes.

A need therefore exists to provide sustained release compositions comprising sustained release beads that comprise coated drug-resin complexes wherein the release of active agent from the beads does not result in dose dumping or high initial burst nor does it result in excessive release retardation or incomplete release of the drug from the composition. The present inventors after rigorous experimentation have found that sustained release compositions comprising sustained release beads that comprise only coated drug-resin complexes comprising drug-resin complexes of at least one active agent and at least one ion-exchange resin; coated with at least one release modifier; wherein the drug-resin complexes are variably coated at different levels of barrier coating of release modifier and wherein the variably coated drug-resin complexes are present in particular proportions, can help achieve sustained release profiles that do not result either in dose dumping or excessive release retardation while providing desired in-vitro drug release profile and bioavailability. The sustained release compositions according to the present invention thus provide release of the active agent at a predetermined rate thereby avoiding too quick a release of the drug and too high peaks of the blood or tissue levels, which can lead to undesirable side effects while also avoiding incomplete release of the drug from the sustained release composition.

### Summary of the Invention

The present invention relates to a sustained release composition comprising a plurality of sustained release beads comprising coated drug-resin complexes comprising:
a. drug-resin complex; and
b. release modifier coating;
wherein the coated drug-resin complexes are present in the form of at least two populations of variably coated drug-resin complexes comprising at least one population of drug-resin complexes coated with at least one release modifier from 1% to 15% by weight of the drug-resin complex; and at least one population of drug-resin complexes coated with at least one release modifier from 15% to 75% by weight of the drug-resin complex and wherein at least two populations of variably coated drug-resin complexes are present in a ratio from 1:9 to 9:1 in the total amount of the coated drug-resin complexes.

### Detailed Description of the Invention

The present invention is set out in the appended set of claims. The embodiments and/or examples of the following description which are not covered by the appended claims are not considered to be part of the present invention.

### Active agents

The term "active agent/s", as employed herein refers to any suitable drug that is capable of complexation with an ion exchange resin, and for which controlled release is desired. In general all, including, but not limited to, acidic, basic, or amphoteric drugs, especially those having short biological half-lives in the order of up to about 12 hours are potential candidates in the compositions of the present invention.

Active agents that can be included in the drug-resin complexes of the present invention include, but are not limited to, one or more analgesics such as, but not limited to, aspirin, codeine, morphine, dihydromorphone, oxycodone, hydrocodone and the like; antihistamines such as, but not limited to, dimenhydrinate, diphenhydramine, chlorpheniramine, brompheniramine, dexchlorpheniramine, hydroxyzine, dexbrompheniramine, fexofenadine, terfenadine, cetirizine, levocetirizine and the like; expectorants or mucolytics such as, but not limited to, ambroxol, bromhexine, carbocisteine, domiodol, guaifenesin and the like; anti-tussive agents such as, but not limited to, codeine, dextromethorphan, hydrocodone and the like; decongestants such as, but not limited to, phenylephrine, pseudoephedrine and the like; analeptic agents; anesthetic agents; anti-asthmatics such as, but not limited to, theophylline and the like; anti-arthritic agents; anti-cancer agents; anti-cholinergic agents; anti-convulsant agents such as, but not limited to, phenobarbital sodium, phenytoin sodium, valproate sodium barbiturates, amylobarbitone sodium, butabarbital sodium, secobarbital sodium and the like; anti-depressant agents; antidiabetics; anti-helminthic agents; anti-diarrheal agents; anti-epileptics such as, but not limited to, phenytoin, meprobamate, nitrezepam and the like; anti-hyperlipidemic agents; antihypertensives such as, but not limited to, clonidine, methyldopa; captopril and the like; antihypotensives such as, but not limited to, propranolol, clonidine and the like; anti-infective agents; anti-inflammatory agents; non-steroidal anti-inflammatory agents such as, but not limited to, naproxyn, diclofenac, indomethacin, ibuprofen, sulindac, meclofenamate sodium, tolmetin sodium and the like; anti-emetics such as, but not limited to, metoclopramide and the like; anti-migraine agents; anti-neoplastic agents; anti-tubercular agents; antibiotics such as, but not limited to, tetracyclines and the like; antacids; antiulcer agents; anti-Parkinsonism drugs; anti-pruritic agents; antipsychotic agents; anti-pyretic agents; anti-spasmodics such as, but not limited, atropine, scopolamine and the like; anti-viral agents; anxiolytic agents; appetite suppressants; attention deficit hyperactivity disorder treating agents, cardiovascular agents including, but not limited to, calcium channel blockers, antianginal agents; central nervous system agents; beta-blockers; antiarrhythmic agents; bronchodilators such as, but not limited to, albuterol; central nervous system stimulants; diuretics such as, but not limited to, ethacrynic acid, bendrofluazide and the like; genetic materials; hormonolytics; hypnotics; hypercalcemics; hypoglycemic agents; immunosuppressive agents; antimuscarinics; genitourinary smooth muscle relaxants; beta-agonists; narcotic antagonists; nicotine; nutritional agents; parasympatholytics; peptide drugs; antihemorrhoidals; psychostimulants; psychotropics; mucolytics; sedatives; laxatives; vitamins; sialagogues, steroids; sympathomimetics; tranquilizers; vasodilators such as, but not limited to, nifedipine, papaverine, diltiazem, nicardirine and the like; or combinations thereof. The active agent/s employed in the compositions of the present invention may be in the form of free base or acid or pharmaceutically acceptable salts, prodrugs, active metabolites, polymorphs, solvates, hydrates, enantiomers, optical isomers, tautomers or racemic mixtures thereof.

The term "anti-tussive agents", as employed herein refers to any compound that can be employed for the treatment, management or mitigation of cough, cold, cold-like and/or flu symptoms and the discomfort, pain, headache, fever and general malaise associated therewith. Suitable anti-tussives that can be incorporated in the compositions of the present invention include, but are not limited to, dextromethorphan, diphenhydramine, caramiphen, carbapentane, ethylmorphine, noscapine, codeine, hydrocodone, and the like or combinations thereof. Furthermore the anti-tussive drugs that are suitable for use in the preparations of the present invention are acidic, amphoteric or basic in nature. The anti-tussive agents employed in the compositions of the present invention may be in the form of free base or acid or pharmaceutically acceptable salts, prodrugs, active metabolites, polymorphs, solvates, hydrates, enantiomers, optical isomers, tautomers or racemic mixtures thereof.

In one embodiment the active agent employed in the composition is an anti-tussive agent. In another embodiment the anti-tussive agent employed in the present invention is dextromethorphan in the form of free base or its pharmaceutically acceptable salts, prodrugs, polymorphs, solvates, hydrates, active metabolites, enantiomers, optical isomers, tautomers or racemic mixtures. In one embodiment, the anti-tussive agent employed in the compositions of the present invention is dextromethorphan hydrobromide.

Further suitable antihistamines that can be incorporated in the compositions of the present invention include, but are not limited to, dimenhydrinate, diphenhydramine, chlorpheniramine, brompheniramine, dexchlorpheniramine, hydroxyzine, dexbrompheniramine, fexofenadine, terfenadine, cetirizine, levocetirizine and the like or combinations thereof. Furthermore the antihistamines suitable for use in the preparations of the present invention are acidic, amphoteric or basic in nature. The antihistamines employed in the compositions of the present invention may be in the form of free base or acid or pharmaceutically acceptable salts, prodrugs, active metabolites, polymorphs, solvates, hydrates, enantiomers, optical isomers, tautomers or racemic mixtures thereof.

In one embodiment the active agent delivered by the sustained release composition of the present invention include, but are not limited to, dextromethorphan, hydrocodone, chlorpheniramine, phenylephrine, pseudoephedrine, codeine, morphine, dihydromorphone, oxycodone, dimenhydrinate, diphenhydramine, brompheniramine, cetirizine, levocetirizine, ambroxol, bromhexine, carbocisteine, domiodol, guaifenesin, hydroxyzine, dexbrompheniramine, fexofenadine, terfenadine, dexchlorpheniramine or combinations thereof in the form of free base, free acid, pharmaceutically acceptable salt, prodrug, active metabolite, polymorph, solvate, hydrate, enantiomer, optical isomer, tautomer or racemic mixture thereof.

Pharmaceutically effective amount of active agent is employed in the composition of the present invention. The term "effective amount" refers to an amount effective to achieve desired preventive, therapeutic and/or beneficial effect. In one embodiment the amount of active agent in the composition can vary from about 0.01 weight % to about 85 weight %, based on the total weight of the composition. In another embodiment the amount of active agent in the composition can vary from about 0.02 weight % to about 75 weight %, based on the total weight of the composition. In still another embodiment, the amount of active agent in the composition can vary from about 0.05 weight % to about 60 weight %, based on the total weight of the composition. In one embodiment the compositions of the present invention may be administered at a dose of about 0.01 mg to about 200 mg of anti-tussive agent or antihistamine. In another embodiment the compositions of the present invention may be administered at a dose of about 0.1 mg to about 150 mg of anti-tussive agent or antihistamine. In still another embodiment the compositions of the present invention may be administered at a dose of about 0.5 mg to about 100 mg of anti-tussive agent or antihistamine. In one embodiment the compositions of the present invention may be administered at a dose of about 0.5 mg to about 100 mg of dextromethorphan hydrobromide. In another embodiment the dose of dextromethorphan hydrobromide is 30 mg. In another embodiment the compositions of the present invention may be administered at a dose of about 0.5 mg to about 100 mg of chlorpheniramine maleate. In another embodiment the dose of chlorpheniramine maleate is 8 mg. In a further embodiment the compositions of the present invention may be administered at a dose of about 0.5 mg to about 100 mg of hydrocodone bitartarate. In another embodiment the dose of hydrocodone bitartarate is 10 mg.

### Ion exchange resins

Active agent employed in the compositions of the present invention is complexed with at least one ion exchange resin. Ion exchange resins suitable for compositions of the present invention comprise a pharmacologically inert organic and/or inorganic matrix containing functional groups that are ionic or capable of being ionized under the appropriate conditions of pH. The organic matrix may be synthetic such as, but not limited to, polymers or copolymers of acrylic acid, methacrylic acid, sulfonated styrene, sulfonated divinylbenzene; or partially synthetic such as, but not limited to, modified cellulose and dextrans. The inorganic matrix includes, but is not limited to, silica gel modified by the addition of ionic groups. Covalently bound ionic groups may be strongly acidic (e.g., sulfonic acid, phosphoric acid), weakly acidic (e.g., carboxylic acid), strongly basic (e.g., primary amine), weakly basic (e.g. quaternary ammonium), or a combination of acidic and basic groups. The ion exchange resin having the polymeric matrix with an anionic functional group is a cation exchange resin and that having a cationic functional group is an anionic exchange resin. The mobile or exchangeable moieties depending on the type of resin can be but not limiting to sodium, hydrogen, potassium, chloride and the like.

In one embodiment of the present invention ion exchange resin employed is a cation exchange resin or an anion exchange resin or combination thereof. In a further embodiment, cation exchange resin is employed for complexation with the active agent. Non limiting examples of suitable cation exchange resin that may be employed include a copolymer of methacrylic acid and divinylbenzene, sodium polystyrene sulfonate resin, sulfonated copolymer of styrene and divinylbenzene, crosslinked polyacrylic acid resin, polyacrylate resin, crosslinked carboxylic acid resin, crosslinked sulfonic acid resin, crosslinked phosphonic acid resin, zeolite or a combination thereof.

In another embodiment, cation exchange resin that may be employed include, but are not limited to, Amberlite® IRP64 (porous copolymer of methacrylic acid and divinylbenzene), Amberlite® IRP69 (sodium polystyrene sulfonate or sulfonated copolymer of styrene and divinylbenzene), Amberlite® IRP88 (cross linked polymer of methacrylic acid and divinylbenzene), DOWEX® RTM. resins (strong cationic exchangers based upon polystyrenesulphonic acid with variable crosslinking (1-12% divinylbenzene)), Tulsion® 335 - (Polacrilex/{Polacirilex S), Tulsion® 339 (Polacrilin potassium USP), Tulsion® 344 (Sodium polystyrene sulfonate BP), Indion® 204 (crosslinked polyacrylic acid), Indion® 214 (crosslinked polyacrylic acid), Indion® 234 (crosslinked polyacrylic acid), Indion® 234S (crosslinked polyacrylic acid), Indion® 294 (crosslinked polyacrylic acid), Purolite® C115 HMR (carboxylic acid functional group), Purolite® C115 E (carboxylic acid functional group), Purolite® C100 HMR (sulfonic acid functional group), Purolite® 100 MR (sulfonic acid functional group), polyacrylate resins, cation exchange resins having phosphonic functional groups or zeolites. Cationic exchange resins are selected for use with basic active agents and molecules having a cationic functionality.

Other suitable ion-exchange resins include anion exchange resins, such as have been described in the art and are commercially available.

The size of the ion-exchange particles that may be employed in the compositions of the present invention may be from about 5 microns to about 750 microns. In one aspect the particle size is within the range of about 40 microns to about 250 microns for liquid dosage forms although particles up to about 1,000 micron can be used for solid dosage forms, e.g., tables and capsules. Both regularly and irregularly shaped resin particles may be employed in the present invention. Regularly shaped particles are those particles that substantially conform to geometric shapes such as spherical, elliptical, and cylindrical and the like, which are exemplified by Dow XYS-40010.00 and Dow XYS-40013.00 (The Dow Chemical Company). Irregularly shaped particles are all particles not considered to be regularly shaped, such as particles with amorphous shapes and particles with increased surface areas due to surface channels or distortions. Irregularly shaped ion-exchange resins of this type are exemplified by Amberlite IRP-69 (Rohm and Haas).

In one embodiment, the ion exchange resin used in the compositions of the present invention is sodium polystyrene sulfonate.

### Drug-resin complexes

Drug-resin complexes according to the present invention comprise at least one active agent and at least one ion-exchange resin. In one embodiment basic active agent is complexed with cation exchange resin. In one embodiment anti-tussive agent is complexed with ion-exchange resin. In another embodiment basic anti-tussive agent is complexed with cation exchange resin. In still another embodiment dextromethorphan hydrobromide is complexed with a cation exchange resin. In another embodiment, anti-tussive agent is complexed with sodium polystyrene sulfonate. In another embodiment dextromethorphan hydrobromide is complexed with sodium polystyrene sulfonate. In a still another embodiment active agent can be complexed with ion exchange resin in any ratio. In a further embodiment, ion exchange resin can be used for complexation with active agent in a ratio of active agent to resin of about 1:0.1 to about 1:20. In another embodiment, ion exchange resin can be used for complexation with active agent in a ratio of active agent to resin of about 1:0.25 to about 1:10. In still another embodiment, ion exchange resin can be used for complexation with active agent in a ratio of active agent to resin of about 1:0.5 to about 1:5.

The drug-ion exchange resin complexes or drug-resin complexes can be prepared using methods known in the art, such as, but not limiting to, blending, slurrying, kneading, grinding, sieving, filling, compressing, lyophilization, spray-drying, fluid-bed drying or centrifugal granulation. The drug-resin binding may be performed, for example, as a batch or column process, as is known in the art. In one illustrative embodiment, drug-resin complex is prepared by batch process. In one embodiment the drug-resin complexes were prepared by stirring aqueous slurry of drug and ion exchange resin for about 0.5 hours to about 12 hours, followed by filtration and drying of the formed drug-resin complex.

In one embodiment, the invention relates to compositions comprising drug-resin complexes having one or more active agents. In another embodiment, the invention also relates to pharmaceutical compositions comprising drug-resin complexes wherein at least one pharmaceutically acceptable excipient has been employed during the process of preparation of the drug-resin complexes, such as, but not limited to, stabilizers and the like to inhibit or prevent degradation of the drug-resin complex during manufacturing process and over shelf life of the composition. Suitable stabilizers include, but are not limited to, antioxidants, chelating agents or combinations thereof. In one embodiment, stabilizer employed during the process of preparation of the drug-resin complexes is an antioxidant. Any suitable antioxidant agent available to those of ordinary skill in the art may be used. Antioxidant such as, but not limited to ascorbic acid, sodium metabisulphite, potassium metabisulfite, sodium bisulfite, sodium sulfite, tocopherol, sorbic acid, retinol, butylated hydroxyanisole (BHA), butylated hydroxytoluene (BHT), propyl gallate, sodium benzoate or any salt thereof, or a combination thereof may be employed. Any suitable chelating agent known to those of ordinary skill in the art may be used. Chelating agents such as, but not limited to, ethylene diaminetetraacetic acid (EDTA), desferrioxamine B, deferoxamine, dithiocarb sodium, penicillamine, pentetate calcium, a sodium salt of pentetic acid, succimer, trientine, nitrilotriacetic acid, trans-diaminocyclohexanetetraacetic acid (DCTA), diethylenetriaminepentaacetic acid, bis(aminoethyl)glycolether-N,N,N',N'-tetraacetic acid, iminodiacetic acid, citric acid, tartaric acid, fumaric acid, or any salt thereof, or a combination thereof may be employed. The stabilizing agent can be present in a concentration of about 0.001 % to 20% by weight of the composition and may or may not be in the final composition.

Further, the drug-resin complexes are impregnated with a solvating agent. The solvating agent can be added as an ingredient in the resin drug complexation step or the drug-resin complexes can be treated with the solvating agent after complexing. This treatment helps particles retain their geometry, and enables the effective application of barrier coatings to the drug-resin complexes resulting in the ability to effectively prolong the release of drugs from drug resin complexes. Solvating agent that can be employed in the compositions of the present invention include, but are not limited to, polyethylene glycol, propylene glycol, mannitol, lactose, methylcellulose, hydroxypropylmethylcellulose, sorbitol, polyvinylpyrrolidone, carboxypolymethylene, xanthan gum, propylene glycol alginate and combinations thereof. In one embodiment the solvating agent is polyethylene glycol. In one embodiment, the drug-resin complexes may not be impregnated with a solvating agent.

In an embodiment of the present invention along with the solvating agent, at least one or more pharmaceutically acceptable excipients, such as but not limited to stabilizers may be employed for impregnation of the drug-resin complexes. The stabilizers that may be employed during impregnation of the drug-resin complexes include the ones as described above under drug-resin complexes.

### Release modifier coating

The release modifier coating comprises at least one release modifier. The drug-resin complexes or impregnated drug-resin complexes are coated with a diffusion barrier coating of at least one release modifier. The release modifiers that may be employed in the compositions of the present invention include, but are not limited to, water-insoluble release modifiers or water-soluble release modifiers or combinations thereof. The water-insoluble release modifiers that may be employed include polymeric water-insoluble release modifier or non-polymeric water-insoluble release modifier or combinations thereof.

Suitable polymeric water-insoluble release modifiers include, but are not limited to, polyvinyl acetate, polyvinyl chloride, polyvinyl carbonate, ethyl cellulose, nitrocellulose, vinylidene chloride-acrylonitrile copolymer, acrylonitrile-styrene copolymer, ethylene vinyl acetate, cellulose acetate, cellulose acetate phthalate, cellulose acetate butyrate, copolymers of vinyl pyrrolidone, blend of polymers comprising polyvinyl acetate, hydroxypropylmethylcellulose phthalate, methacrylic acid copolymers such as Eudragit® LI00/SI00/LI00-55 and the like or mixtures thereof; methacrylate copolymers such as Eudragit® E100/EPO, Eudragit® RL100/RL30D/RLPO, Eudragit® RS100/RS30D/RSPO and the like or mixtures thereof.

Suitable non-polymeric water-insoluble release modifiers include, but are not limited to, fats, oils, waxes, fatty acids, fatty acid esters, glycerides, long chain monohydric alcohols and their esters, phospholipids, terpenes or combinations thereof. The non-polymeric water-insoluble release modifiers employed in the compostions of the present invention include, but are not limited to, Cutina® (hydrogenated castor oil), Hydrobase® (hydrogenated soybean oil), Castorwax® (hydrogenated castor oil), Croduret® (hydrogenated castor oil), Carbowax®, Compritol® (glyceryl behenate), Sterotex® (hydrogenated cottonseed oil), Lubritab® (hydrogenated cottonseed oil), Apifil® (wax yellow), Akofine® (hydrogenated cottonseed oil), Softisan® (hydrogenated palm oil), Hydrocote® (hydrogenated soybean oil), Corona® (Lanolin), Gelucire® (macrogolglycerides Lauriques), Precirol® (glyceryl palmitostearate), Emulcire™ (cetyl alcohol), Plurol® diisostearique (polyglyceryl diisostearate), Geleol® (glyceryl stearate),and mixtures thereof. In another embodiment, lipids or waxes can also be employed in the form of an aqueous dispersion stabilized by surfactants and suitable stabilizers.

Suitable water soluble release modifiers that may be employed include, but are not limited to, polyvinylpyrrolidone, poloxamer, guar gum, xanthan gum, gum arabic, tragacanthan, cellulose derivatives such as hydroxypropylmethylcellulose, hydroxypropyl cellulose, methylcellulose, and hydroxyethyl cellulose, carboxymethylethyl cellulose, hydroxyethylmethyl carboxymethyl cellulose, hydroxyethyl methyl cellulose, carboxymethyl cellulose, methylhydroxyethyl cellulose, methylhydroxypropyl cellulose or any mixtures thereof.

In one embodiment the release modifier employed is ethyl cellulose.

The release modifiers of the present invention may be used in admixture with at least one pharmaceutically acceptable excipient, such as but not limited to, plasticizers, pigments and the like or any mixtures thereof. Suitable plasticizers include, but are not limited to, dibutyl sebacate, propylene glycol, polyethylene glycol, polyvinyl alcohol, triethyl citrate, acetyl triethyl citrate, acetyl tributyl citrate, tributyl citrate, triacetin or the like or any combinations thereof. In an embodiment, the drug-resin complexes are directly coated with the water-insoluble release modifier.

A coating procedure known to a person skilled in the art, which provides a substantially complete coating on the impregnated drug-resin complexes without significant agglomeration of the drug-resin complex particles, may be used. Coating to the drug-resin complexes may be applied using wet granulation, dry granulation, melt granulation, melt coating, physical mixing, spray coating and the like. Coatings may be applied in a coating pan or with a fluid-bed coating apparatus. The release modifier coatings may be applied from aqueous suspension or organic solvents. Optionally after coating the coated drug-resin complexes may be cured at a suitable temperature and for a suitable amount of time. In one embodiment, only coated drug-resin complexes are incorporated in the compositions of the present invention. Optimum coat weight and coat thickness may be determined for each drug-resin complex and generally depends on the drug release characteristics of the resin for that particular active agent. In one embodiment the drug-resin complexes are variably coated at different levels of barrier coating of release modifier and the variably coated drug-resin complexes are present in particular proportions in the sustained release compositions. The presence of such variably coated drug-resin complexes helps achieve the desired release profiles that does not result either in dose dumping or excessive release retardation. The compositions of the present invention comprise at least two variably coated populations or portions of coated drug-resin complexes or the coated drug-resin complexes are present in the form of at least two populations of variably coated drug-resin complexes.

In the present invention, the variably coated drug-resin complexes comprise at least one population of drug-resin complexes coated with at least one release modifier from about 1% to about 15% by weight of the drug-resin complex; and at least one population of drug-resin complexes coated with at least one release modifier from about 15% to about 75% by weight of the drug-resin complex. In another embodiment drug-resin complexes coated with a barrier coating of at least one release modifier from about 1% to 15% by weight of the drug resin complex and the drug-resin complexes coated with a barrier coating of at least of release modifier to not less than about 15% by weight and not more than 75% by weight are present in a ratio from about 1:9 to about 9:1 in the total amount of the coated drug-resin complexes employed in the compositions of the present invention. In a further embodiment drug-resin complexes coated with a barrier coating of at least one release modifier from about 1% to 10% by weight of the drug resin complex and the drug-resin complexes coated with a barrier coating of at least of release modifier to not less than about 20% by weight and not more than 75% by weight are present in a ratio from about 1:9 to about 9:1 in the total amount of the coated drug-resin complexes employed in the compositions of the present invention. The sustained release composition comprising plurality of sustained release beads comprising coated drug-resin complexes comprising: (a) drug-resin complex comprising at least one active agent and at least one ion-exchange resin; and (b) release modifier coating; wherein coated drug-resin complexes are present in the form of at least two populations of variably coated drug-resin complexes comprising at least one population of drug-resin complexes coated with at least one release modifier from about 1% to about 15% by weight of the drug-resin complex; and at least one population of drug-resin complexes coated with at least one release modifier from about 15% to about 75% by weight of the drug-resin complex.

The coated drug-resin complexes may be present in the sustained release beads of the present invention in an amount of from about 20% to about 100% by weight of the beads.

### Sustained release beads

Sustained release beads of the present invention comprise the coated drug-resin complexes discussed above. In one embodiment the coated drug-resin complexes are the sustained release beads. In another embodiment the sustained release beads comprise coated drug-resin complexes and at least one pharmaceutically acceptable excipient such as, but not limited to, diluents, stabilizers, release modifiers, or the like or any combinations thereof. In one embodiment the sustained release beads of the present invention comprise variably coated drug-resin complexes in particular proportions as discussed under coated drug-resin complexes above. The compositions of the present invention comprise one or more sustained release beads. The sustained release beads employed in the present invention are in forms such as, but not limited to, powder, particles, granules, pellets, beads, minitablets, tablets and the like or any combinations thereof. The sustained release beads may be present in the compositions in an amount from about 5% to about 95% by weight of the composition. In a further embodiment the coated drug-resin complexes and/or sustained beads may be overcoated using conventional polymers including, but not limited to, hydroxypropyl methyl cellulose, hydroxypropyl cellulose, carboxymethyl cellulose, polyvinyl alcohol polymethacrylates and the like or combinations thereof, waxes, and combinations thereof; or using release modifiers listed above under release modifiers. In one embodiment sustained release beads in the form of drug-resin complexes are incorporated in the sustained release pharmaceutical compositions of the present invention. The sustained release beads are incorporated in the sustained release pharmaceutical compositions of the present invention by any of the methods generally known to a person skilled in the art, especially depending on the form of the sustained release beads being incorporated and the final form of the pharmaceutical composition.

### Sustained release compositions

Sustained release pharmaceutical compositions of the present invention comprise plurality of sustained release beads and at least one pharmaceutically acceptable excipient. The sustained release compositions may be formulated for delivery of active agent by any suitable route including, e.g. orally, topically, intraperitoneally, transdermally, sublingually, intramuscularly, transmucosally, rectally, subcutanoeulsly, transnasally or via inhalation. In one embodiment, the sustained release compositions are for oral delivery. The compositions for oral delivery may be in any form, such as, but not limited to, liquid, solid or semi-solid preparations and the like. Liquid preparations for oral administration may be in any form including, but not limited to, suspensions, syrups or the like. Solid preparations for oral administration may be in any form including, but not limited to, capsules, tablets, caplets, orally disintegrating tablets, dispersible tablets, dry suspension for reconstitution, granules, wafers, bite-dispersion tablets and the like or any combinations thereof. In one embodiment the sustained release preparation of the present invention is a suspension.

The sustained release compositions of the present invention comprise at least one pharmaceutically acceptable excipient, depending on the final dosage form to be prepared, such as, but not limited to, binders, disintegrants, superdisintegrants, diluents, salivating agents, surfactants, flavors, sweeteners, colorants, souring agents, viscolizers, glidants, lubricants, solubilizers, stabilizers, suspending agents, preservatives, cosolvents, anti-caking agents, buffers and the like or any combinations thereof.

Suitable disintegrants can be selected from, but not limiting to, crospovidone, calcium silicate and starch. Suitable superdisintegrants include, but are not limited to, natural, modified or pregelatinized starch, crospovidone, croscarmellose sodium, sodium starch glycolate, low-substituted hydroxypropyl cellulose. Examples of suitable binders include, but are not limited to, starch, pregelatinized starch, polyvinyl pyrrolidone, copovidone, cellulose derivatives, such as hydroxypropylmethyl cellulose, hydroxypropyl cellulose and carboxymethyl cellulose and their salts. Examples of suitable diluents include, but are not limited to, starch, microcrystalline cellulose, lactose, xylitol, mannitol, maltose, polyols, fructose, guar gum, sorbitol, magnesium hydroxide, dicalcium phosphate, coprocessed mannitol and calcium silicate and the like or any combinations thereof. Examples of lubricants include, but are not limited to, magnesium stearate, calcium stearate, stearic acid, talc, and sodium stearyl fumarate. Suitable glidants includes but are not limited to, colloidal silica, silica gel, precipitated silica, or combinations thereof. Suitable salivating agents include, but are not limited to, micronised polyethylene glycol, sodium chloride or precipitated micronised silica. Examples of solubilizers include, but are not limited to cetostearyl alcohol, cholesterol, diethanolamine, ethyl oleate, ethylene glycol palmitostearate, glycerin, glyceryl monostearate, isopropyl myristate, lecithin, medium-chain glyceride, monoethanolamine, oleic acid, propylene glycol, polyoxyethylene alkyl ether, polyoxyethylene castor oil glycoside, polyethylene sorbitan fatty acid ester, polyoxyethylene stearate, propylene glycol alginate, sorbitan fatty acid ester, stearic acid, sunflower oil, triethanolmine,or combinations thereof. Souring agents include, but are not limited to, monosodium fumarate and/or citric acid. The compositions of the present invention may also include stabilizers such as, but not limited to, those described above under drug-resin complexes.

Suitable viscolizers include, but are not limited to, coprocessed microcrystalline cellulose such as but not limited to, Avicel RC591, Avicel CL-611, D-sorbitol solution, polyalkylene oxides such as, but not limited to polyethylene oxide; cellulose ethers such as, but not limited to hydroxyethyl cellulose, hydroxypropylcellulose, hydroxypropyl methyl cellulose, methyl cellulose, ethyl cellulose, sodium carboxy methylcellulose, calcium carboxymethyl cellulose, microcrystalline cellulose; gums such as but not limited to gum arabic alginates, agar, sodium alginate guar gum, locust bean, carrageenan, tara, gum arabic, tragacanth, pectin, xanthan, gellan, maltodextrin, galactomannan, pusstulan, laminarin, scleroglucan, gum arabic, inulin, karaya, whelan; polyols such as, but not limited to dipropylene glycol, polypropylene glycol, propylene glycol, polyethylene glycol (PEG), sorbitol and glycerol; carbopol, starch and starch-based polymers such as, but not limited to, pregelatinized starch, acrylic acid and methacrylic acid polymers, and esters thereof, maleic anhydride polymers; polymaleic acid; poly(acrylamides); poly(olefinic alcohol)s; poly(N-vinyl lactams); polyoxyethylated saccharides; polyoxazolines; polyvinylamines; polyvinylacetates; polyimines; povidone, vinylpyrrolidone/vinyl acetate copolymer and polyvinyl acetate, mixture of polyvinyl acetate and polyvinylpyrrolidone, chitin, cyclodextrin, gelatin, chitosan and the like or any mixtures thereof.

Suitable surfactants include, but are not limited to, anionic, nonionic, cationic, and zwitterionic surfactants or a mixture thereof. The non-ionic surfactants employed in the composition may include, but are not limited to, ethoxylated fatty acid ester, ethoxylated fatty acid ethers, ethoxylated sorbitan ethers, ethoxylated alkyl-phenols, glycerol esters, glycerol sugar esters, polyoxyethyleneglycerol monolaurate, polyoxyethyleneglycerol monostearate, polyoxyethylene-20-cetyl stearate, polyoxyethylene-25-cetyl stearate, polyoxyethylene (25)-oxypropylene monostearate, polyoxyethylene-20-sorbitan monopalmitate, poly-oxyethylene-16-tert-octylphenol, polyoxyethylene-20-cetyl ether, polyethylene glycol(1000) monocetyl ether, ethoxylated castor oil, polyoxyethylene sorbitol-lanolin derivatives, polyoxyethylene(25)propylene glycol stearate, polyoxyethylenesorbitol esters, polyoxyethylene-20-sorbitan monopalmitate, polyoxyethylene-16-tert-octylphenol, polyoxyethylene-20-cetyl ether, glycyeryl undecylenate and Polysorbate 60, capmul (medium chain glyceride),peceol (glyceryl monooleate), glyceryl laurate and glyceryl caprylate (Capmul MCM), PEG sorbitan fatty acid esters like PEG-20 sorbitan monolaurate (Tween 20), PEG-20 sorbitan monostearate (Tween 60), PEG-20 sorbitan monooleate (Tween 80), sorbitan fatty acid esters like sorbitan monolaurate (Span 20), glyceryl stearate (Cithrol GMS) or the like and mixtures thereof. Suitable cationic surfactants include, but are not limited to, quaternary ammonium compounds, alkylamidoamines and quaternary ester compounds, distearyl dimethyl ammonium chloride, dimyristyl dimethyl ammonium chloride, dipalmityl dimethyl ammonium chloride or the like and mixtures thereof. Suitable anionic surfactants include, but are not limited to, fatty alcohol sulfates, alpha olefin sulfonates, sulfosuccinates, phosphate esters, carboxylates, sarcosinates, alkyl benzene sulfonates, alkyl sulfonates, olefin sulfonates, alkyl ethersulfonates, glycerol ethersulfonates, α-methyl estersulfonates, sulfonic fatty acids, alkyl sulfates, fatty alcohol ethersulfates, glycerol ethersulfates, mixed hydroxy ethersulfates, monoglyceride (ether)sulfates, fatty acid amide (ether)sulfates, sulfosuccinates, sulfosuccinamates, sulfotriglycerides, amide soaps, ether carboxylic acids, isethionates, sarcosinates, taurides, alkyl oligoglycoside sulfates, alkyl (ether)phosphates or the like and mixtures thereof. Suitable zwitterionic surfactants employed include, but are not limited to, N-alkyl-N,N-dimethyl ammonium glycinates, for example cocoalkyl dimethyl ammonium glycinate, N-acyl aminopropyl-N,N-dimethyl ammonium glycinates, cocoacyl aminoethyl hydroxyethyl carboxymethyl glycinate or the like and mixtures thereof.

Further, the composition of the present invention may further comprise a preservative such as but not limited to methyl parahydroxybenzoate, propyl parahydroxybenzoate and sodium benzoate. Suitable cosolvent that may be used includes, but is not limited to, ethanol and polyhydric alcohols such as, but not limited to, glycerin, propylene glycol, low molecular weight polyethylene glycols, and mixtures thereof. Further anti-caking agents that may be optionally incorporated include, but are not limited to, colloidal silicon dioxide, tribasic calcium phosphate, powdered cellulose, magnesium trisilicate, starch, and mixtures thereof. Suitable sweetening agent includes, but is not limited to, aspartame, stevia extract, glycyrrhiza, saccharine, saccharine sodium, acesulfame, sucralose, dipotassium glycyrrhizinate, galactose, fructose, high fructose corn syrup, dextrose, sucrose, sugar, maltose, partially hydrolyzed starch, corn syrup solids, sorbitol, xylitol, mannitol and the like or mixtures thereof. The compositions may comprise one or more natural and/or artificial flavors such as, but not limited to, mint flavour, orange flavour, lemon flavors, strawberry aroma, vanilla flavour, raspberry aroma, cherry flavor, tutty frutty flavor, magnasweet 135, key lime flavor, grape flavor, trusil art 511815, and fruit extracts and the like. Suitable colorants include, but are not limited to, pigments and dyes such as FD&C Red, FD&C Yellow, FD&C Green, and FD&C Blue and the like or combinations thereof.

In one of the embodiment, the solid dosage form of the present invention may be optionally coated. Surface coating may be employed for aesthetic purposes or for dimensionally stabilizing the compressed dosage form. The coating may be carried out using any conventional technique employing conventional ingredients suitable for oral use. A surface coating can, for example, be in the form of a film using conventional polymers including, but not limited to, hydroxypropyl methyl cellulose, hydroxypropyl cellulose, carboxymethyl cellulose, polyvinyl alcohol polymethacrylates and the like, and combinations thereof. In another embodiment of the present invention, the composition may be optionally coated with a functional coat. The functional coat may be applied using coating agents including, but not limited to, hydrophilic polymers, hydrophobic polymers, waxes, and the like, or mixtures thereof, either alone or in combination, along with plasticizers, colorants, opacifiers etc. The functional coat may help provide the desired drug release profile.

The sustained release compositions of the present invention can be readily formulated according to methods well known to those skilled in the art. Method of preparation of the compositions of the present invention depends on the final dosage form desired.

The compositions of the present invention provide sustained release of the active agent in-vitro and in-vivo for up to about 24 hours. In one embodiment the compositions of the present invention provide sustained release of the active agent in-vitro and in-vivo for up to about 12 hours. In another embodiment the compositions of the present invention provide sustained release of the anti-tussive agent in-vitro and in-vivo for up to about 12 hours. In another embodiment, the compositions of the present invention provide sustained release of the anti-tussive agent in-vitro and in-vivo for about 6 to about 12 hours. In yet another embodiment, the compositions of the present invention provide sustained release of the anti-tussive agent in-vitro and in-vivo for up to about 24 hours. In still another embodiment, the compositions of the present invention provide sustained release of the anti-tussive agent in-vitro and in-vivo for about 6 to about 24 hours.

In a further embodiment is provided use of the sustained release compositions of the present invention for the prevention, treatment, management or mitigation of various disease conditions or disorders depending on the active agent employed. In one embodiment is provided use of the sustained release compositions of the present invention for the treatment, management or mitigation of cough, cold, cold-like and/or flu symptoms and the discomfort, pain, headache, fever and general malaise associated therewith. In another embodiment is provided use of the sustained release compositions of the present invention for the manufacture of a medicament for the prevention, treatment, management or mitigation of various disease conditions or disorders depending on the active agent employed. In a still further embodiment is provided use of the sustained release compositions of the present invention for the manufacture of a medicament for the treatment, management or mitigation of cough, cold, cold-like and/or flu symptoms and the discomfort, pain, headache, fever and general malaise associated therewith. In another embodiment, a method of treating, managing or mitigating cough, cold, cold-like and/or flu symptoms and the discomfort, pain, headache, fever and general malaise associated therewith is provided which comprises administering to the subject in need thereof sustained release composition of the present invention.

In another embodiment, the invention also relates to sustained release compositions comprising at least one second or additional active agent in addition to at least one active agent present in the compositions. In one embodiment, the second or additional active agent is for immediate release. In another embodiment, the second or additional active agent is different than the first active agent that is delivered in a sustained manner. In still another embodiment the second or additional active agent is complexed with ion-exchange resin. In another embodiment the second active agent is not complexed with ion exchange resin. In a further embodiment the second active agent or additional active agent is delivered in a sustained release manner. Such a second or additional active agent includes, but is not limited to, the list of active agents discussed above under active agents.

### EXAMPLES

### Example 1: Comparative evaluation of drug release from sustained release dextromethorphan compositions

Comparative evaluation of three sustained release dextromethorphan compositions was carried out. The three compositions being evaluated comprised:
A) Coated drug-resin complexes; wherein all the drug-resin complexes are coated at a single coating level of 25% by weight of the drug-resin complex.
B) Coated drug-resin complexes of A and uncoated drug-resin complexes in a ratio of 2:1.
C) Coated drug resin complexes of the present invention that are variably coated and as per an embodiment a portion of drug-resin complexes are coated at one coating level of 5% by weight of the drug-resin complex while another portion of drug-resin complexes are coated at another coating level of 25% by weight of the drug-resin complex and present in a ratio of 1:2 respectively.

**Table 1: Compositions of dextromethorphan sustained release beads**

| **Composition** | **A*** | **B*** | **C** |
|---|---|---|---|
| **Ingredients** | **%w/w** | **%w/w** | **%w/w** |
| Dextromethorphan HBr | 0.6 | 0.6 | 0.6 |
| Sodium polystyrene sulfonate, USP | 1.8 | 1.8 | 1.8 |
| Polyethylene glycol, USPNF | 0.64 | 0.43 | 0.64 |

| Release modifier coating | | | |
|---|---|---|---|
| Ethyl cellulose, USP | 0.63 | 0.42 | 0.43 |
| Dibutyl sebacate, USPNF | 0.19 | 0.12 | 0.13 |
| Isopropyl alcohol | 8.43 | 5.62 | 5.79 |
| Water | q.s. | q.s. | q.s. |

| | | | |
|---|---|---|---|
| *Comparative Examples, not being part of the Invention. | | | |

Procedure: Dextromethorphan HBr was complexed using ion exchange resin in water under stirring. The drug-resin complex formed was filtered, dried and solvated. This treated drug-resin complex was then coated with ethyl cellulose to a coating level of 5% weight gain and 25 % weight gain. A separate portion of drug-resin complex was not coated with ethyl cellulose. These beads were then incorporated into the following suspension base.

**Table 2: Composition of suspension base**

| **Ingredients** | **%w/w** |
|---|---|
| Pharma grade sugar | 35.0 |
| Methylparaben, USPNF | 0.1 |
| Propylparaben, USPNF | 0.01 |
| Disodium EDTA, USPNF | 0.005 |
| Xanthan gum, USPNF | 0.10 |
| High fructose corn syrup, USPNF | 12.0 |
| F D&C Yellow | 0.02 |
| Natural orange flavor | 0.18 |
| Polyoxyethylene sorbitan fatty acid esters, USP | 0.1 |
| Citric acid, USP | 0.25 |
| Purified water | q.s to 100 |

Procedure: Methylparaben, propylparaben, disodium EDTA were dissolved in purified water at 85-90°C. Pharma grade sugar was added to above solution under stirring and solution was subsequently cooled to room temperature. Xanthan gum was dispersed in high fructose corn syrup under stirring. This dispersion was added to the above sugar syrup under stirring to get uniform dispersion. Solution of color and flavor was then added. The beads were then added to the syrup base along with polyoxyethylene sorbitan fatty acid esters and citric acid. Final volume was adjusted with purified water.

For composition "A" only 25% coated drug-resin complexes were added in suspension base. For composition "B" combination of 25% coated and uncoated drug-resin complexes in ratio of 2:1 respectively were added in suspension base. For composition "C" combination of 5% coated drug-resin complexes and 25% coated drug-resin complexes in a proportion of 1:2 respectively were added in suspension base.

Comparative dissolution profile of the three compositions was evaluated in pH change media using USP Type II apparatus and 100 rpm speed. The pH change media to be used was 0.1 N HCI for 1 hour followed by pH 6.6 phosphate buffer.

**Table 3: Comparative dissolution profiles of the three compositions**

| **Time (hrs)** | **Compositions** | | |
|---|---|---|---|
| | **A** | **B** | **C** |
| 0 | 0.0 | 0.0 | 0.0 |
| 0.5 | 15.7 | 28.4 | 25.6 |
| 1 | 20.6 | 31.7 | 29.2 |
| 2 | 49.2 | 67.1 | 58.8 |
| 4 | 74.5 | 81.4 | 70.3 |
| 6 | 77.9 | 83.4 | 75.6 |

The comparative study of the three compositions shows that composition "A" shows excessive retardation of drug release while composition "B" shows faster drug release and Composition "C" shows optimal release profile of the drug from the composition that is neither too retarded nor too fast.

### Example 2: Dextromethorphan sustained release suspensions

**Table 4: Composition of dextromethorphan sustained release suspension**

| **Ingredients** | **mg/5ml** |
|---|---|
| Dextromethorphan HBr | 30 |
| Sodium polystyrene sulfonate, USP | 60 |
| Polyethylene glycol, USPNF | 32 |
| Sodium metabisulphite, USPNF | 20 |
| Ethyl cellulose, USP | 21.81 |
| Dibutyl sebacate, USPNF | 6.51 |
| Isopropyl alcohol | 293.25 |
| Pharma grade sugar | 1750 |
| Methylparaben, USPNF | 5 |
| Propylparaben, USPNF | 0.5 |
| High fructose corn syrup | 850 |
| Xanthan gum, USPNF | 5.5 |
| Orange flavor | 9 |
| FD & C Yellow | 0.375 |
| FD & C Red | 0.375 |
| Polyoxyethylene sorbitan fatty acid esters, USP | 5 |
| Citric acid anhydrous, USP | 7.5 |
| Purified water | qs 100 |

Same procedure as for example 1(C) was employed for the preparation of the sustained release preparation.

This dextromethorphan suspension has a desired sustained release profile.

### Example 3: Dextromethorphan sustained release suspensions

**Table 5: Composition of dextromethorphan sustained release suspension**

| **Ingredients** | **mg/5ml** |
|---|---|
| Dextromethorphan HBr | 30 |
| Sodium polystyrene sulfonate, USP | 60 |
| Polyethylene glycol, USPNF | 32 |
| Sodium metabisulphite, USPNF | 5 |
| Ammonio methacrylate copolymer, Type A Ph.Eur | 20 |
| Pharma grade sugar | 1750 |
| Methylparaben, USPNF | 5 |
| Propyl paraben, USPNF | 0.5 |
| High fructose corn syrup | 850 |
| Xanthan gum, USPNF | 5.5 |
| Orange flavor | 9 |
| FD & C Yellow | 0.375 |
| FD & C Red | 0.375 |
| Polyoxyethylene sorbitan fatty acid esters, USP | 10 |
| Citric acid anhydrous, USP | 7.5 |
| Purified water | q.s. 100 |

Same procedure as for Example 1(C) is employed for the preparation of the sustained release preparation

This dextromethorphan suspension has a desired sustained release profile.

### Example 4: Dextromethorphan sustained release orally disintegrating tablets

**Table 6: Composition of dextromethorphan sustained release orally disintegrating tablets**

| **Ingredients** | **mg/tablet** |
|---|---|
| Dextromethorphan HBr | 30 |
| Amberlite IRP 69 | 70 |
| Polyethylene glycol, USPNF | 27 |
| Sodium metabisulphite, USPNF | 20 |
| Ethyl cellulose, USP | 22 |
| Dibutyl sebacate, USPNF | 7 |
| Coprocessed mannitol and calcium silicate | 105 |
| Microcrystalline cellulose, USP | 40 |
| Crospovidone, USP/NF | 18 |
| Aspartame, USP | 6 |
| Colloidal silicon dioxide, USP | 2 |
| Magnesium stearate, USP | 2 |
| Flavor | 1 |
| **Total** | **350** |

Procedure: Sodium metabisulphite, dextromethorphan HBr and Amberlite IRP 69 were added to water under stirring. The drug-resin complex formed was filtered, dried and solvated. This treated drug-resin complex was then coated with ethyl cellulose to a coating level of 5% weight gain and 25 % weight gain. The coated drug-resin complexes were blended with other excipients, lubricated and the lubricated blend was compressed to form orally disintegrating tablets with the following parameters:

| | | |
|---|---|---|
| Hardness (N) | : | 30-40 |
| Friability (%) | : | 0.40 |
| Disintegration time (sec) | : | 15-20 |
| Disintegration time in oral cavity (sec) | : | 40-50 |

Tablets with desired taste-masking, friability and disintegration time were obtained.

## Claims

1. A sustained release composition comprising a plurality of sustained release beads comprising coated drug-resin complexes comprising:
a. drug-resin complex; and
b. release modifier coating;
wherein the coated drug-resin complexes are present in the form of at least two populations of variably coated drug-resin complexes comprising at least one population of drug-resin complexes coated with at least one release modifier from 1% to 15% by weight of the drug-resin complex; and at least one population of drug-resin complexes coated with at least one release modifier from 15% to 75% by weight of the drug-resin complex and wherein at least two populations of variably coated drug-resin complexes are present in a ratio from 1:9 to 9:1 in the total amount of the coated drug-resin complexes.

2. The composition of claim 1 wherein the drug-resin complex comprises at least one active agent and at least one ion-exchange resin.

3. The composition of claim 2 wherein the active agent is an anti-tussive agent, an analgesic, an antihistamine, an expectorant, a mucolytic, a decongestant, an analeptic agent, an anesthetic agent, an anti-asthmatic, an anti-arthritic agent; an anti-cancer agent, an anti-cholinergic agent, an anti-convulsant agent, an anti-depressant agent, an antidiabetic, an anti-helminthic agent, an anti-diarrheal agent; an anti-epileptic, an anti-hyperlipidemic agent; an antihypertensive, an antihypotensive, an anti-infective agent, an anti-inflammatory agent, a non-steroidal anti-inflammatory agent, an anti-emetic, an anti-migraine agent; an anti-neoplastic agent, an anti-tubercular agent, an antibiotic, an antacid, an antiulcer agent; an anti-Parkinsonism drug, an anti-pruritic agent, an antipsychotic agent, an anti-pyretic agent, an anti-spasmodic, an anti-viral agent, an anxiolytic agent, an appetite suppressant, an attention deficit hyperactivity disorder treating agent, a cardiovascular agent, a calcium channel blocker, an antianginal agent, a central nervous system agent, a beta-blocker, an antiarrhythmic agent, a bronchodilator, a central nervous system stimulant, a diuretic, a genetic material, a hormonolytic, a hypnotic, a hypercalcemic, a hypoglycemic agent, an immunosuppressive agent, an antimuscarinic, a genitourinary smooth muscle relaxant, a beta-agonist, a narcotic antagonist, nicotine, a nutritional agent, a parasympatholytic, a peptide drug, an antihemorrhoidal, a psychostimulant, a psychotropic, a mucolytic, a sedative, a laxative, a vitamin, a sialagogue, a steroid, a sympathomimetic, a tranquilizer, or a vasodilator or a combination thereof in the form of free base, free acid, pharmaceutically acceptable salt, solvate or hydrate thereof.

4. The composition of claim 2 wherein the active agent is dextromethorphan, hydrocodone, chlorpheniramine, phenylephrine, pseudoephedrine, codeine, morphine, dihydromorphone, oxycodone, dimenhydrinate, diphenhydramine, brompheniramine, cetirizine, levocetirizine, ambroxol, bromhexine, carbocisteine, domiodol, guaifenesin, hydroxyzine, dexbrompheniramine, fexofenadine, terfenadine, dexchlorpheniramine or combination thereof in the form of free base, free acid, pharmaceutically acceptable salt, solvate or hydrate thereof.

5. The composition of claim 2 wherein the ion exchange resin is a cation exchange resin or an anion exchange resin or combination thereof.

6. The composition of claim 5 wherein the cation exchange resin is a copolymer of methacrylic acid and divinylbenzene, a sodium polystyrene sulfonate resin, a sulfonated copolymer of styrene and divinylbenzene, a crosslinked polyacrylic acid resin, a polyacrylate resin, a crosslinked carboxylic acid resin, a crosslinked sulfonic acid resin, a crosslinked phosphonic acid resin, zeolite or a combination thereof.

7. The composition of claim 2 wherein the active agent and the ion exchange resin are present in a ratio of 1:0.1 to 1:20 in the drug-resin complex.

8. The composition of claim 1 wherein the release modifier coating comprises at least one release modifier, said release modifier being a water-insoluble release modifier, a water-soluble release modifier or a combination thereof; wherein said water soluble release modifier is optionally selected from polyvinylpyrrolidone, poloxamer, guar gum, xanthan gum, gum arabic, tragacanthan, cellulose derivatives such as hydroxypropylmethylcellulose, hydroxypropyl cellulose, methylcellulose, and hydroxyethyl cellulose, carboxymethylethyl cellulose, hydroxyethylmethyl carboxymethyl cellulose, hydroxyethyl methyl cellulose, carboxymethyl cellulose, methylhydroxyethyl cellulose, or methylhydroxypropyl cellulose or a combination thereof.

9. The composition of claim 8 wherein the water-insoluble release modifier is a polymeric water-insoluble release modifier, a non-polymeric water-insoluble release modifier or a combination thereof; wherein said polymeric water-insoluble release modifier is optionally selected from polyvinyl acetate, polyvinyl chloride, polyvinyl carbonate, ethyl cellulose, nitrocellulose, vinylidene chloride-acrylonitrile copolymer, acrylonitrile-styrene copolymer, ethylene vinyl acetate, cellulose acetate, cellulose acetate phthalate, cellulose acetate butyrate, copolymer of vinyl pyrrolidone, hydroxypropylmethylcellulose phthalate, methacrylic acid copolymer, or methacrylate copolymer or a combination thereof.

10. The composition of claim 1 wherein the sustained release beads and the composition further comprise at least one pharmaceutically acceptable excipient, said pharmaceutically acceptable excipient being selected from diluent, stabilizer, release modifier, binder, disintegrant, superdisintegrant, salivating agent, surfactant, flavor, sweetener, colorant, souring agent, viscolizer, glidant, lubricant, solubilizer, stabilizer, suspending agent, preservative, cosolvent, anti-caking agent, or buffer or a combination thereof.

11. The composition of claim 1 wherein the sustained release composition is in the form of a liquid, a solid or a semisolid preparation; said liquid preparation being suspension and said solid preparation being capsule, tablet, caplet, orally disintegrating tablet, dispersible tablet, dry suspension for reconstitution, granule, wafer or bite-dispersion tablet.

12. The composition of claim 1 wherein the sustained release composition further comprises an additional active agent wherein said additional active agent is optionally delivered in an immediate release or sustained release manner.

## Patentansprüche

1. Zusammensetzung mit verzögerter Freisetzung, umfassend eine Vielzahl von Kügelchen mit verzögerter Freisetzung
umfassend beschichtete Arzneimittel-Harz-Komplexe umfassend:
a. Arzneimittel-Harz-Komplex; und
b. Freisetzungsmodifikator-Beschichtung;
wobei die beschichteten Arzneimittel-Harz-Komplexe vorliegen in Form von mindestens zwei Populationen von variabel beschichteten Arzneimittel-Harz-Komplexen umfassend mindestens eine Population von Arzneimittel-Harz-Komplexen mit Beschichtung mit mindestens einem Freisetzungsmodifikator von 1 % bis 15 % nach Gewicht des Arzneimittel-Harz-Komplexes; und mindestens eine Population von Arzneimittel-Harz-Komplexen mit Beschichtung mit mindestens einem Freisetzungsmodifikator von 15 % bis 75 % nach Gewicht des Arzneimittel-Harz-Komplexes und wobei mindestens zwei Populationen von variabel beschichteten Arzneimittel-Harz-Komplexen in einem Verhältnis von 1:9 bis 9:1 in der Gesamtmenge der beschichteten Arzneimittel-Harz-Komplexe vorliegen.

2. Zusammensetzung nach Anspruch 1, wobei der Arzneimittel-Harz-Komplex mindestens einen Wirkstoff und mindestens ein Ionenaustauschharz umfasst.

3. Zusammensetzung nach Anspruch 2, wobei der Wirkstoff ein Antitussivum, ein Analgetikum, ein Antihistamin, ein Expectorans, ein Mukolytikum, ein Dekongestivum, ein Analeptikum, ein Anästhetikum, ein Antiasthmatikum, ein Antiarthritikum; ein Antikrebsmittel, ein Anticholinergikum, ein Antikonvulsivum, ein Antidepressivum, ein Antidiabetikum, ein Antihelminthikum, ein Anti-Diarrhoe-Mittel; ein Antiepileptikum, ein Antihyperlipidämikum; ein Antihypertensivum, ein Antihypotensivum, ein Antiinfektiosum, ein entzündungshemmendes Mittel, ein nicht-steroidales entzündungshemmendes Mittel, ein Antiemetikum, ein Antimigränemittel; ein antineoplastisches Mittel, ein Antituberkelmittel, ein Antibiotikum, ein Antazidum, ein Anti-Ulcus-Mittel; ein Antiparkinsonmittel, ein antipruriginöses Mittel, ein Antipsychotikum, ein Antipyretikum, ein Antispasmodikum, ein Anti-Virus-Mittel, ein Anxiolytikum, ein Appetitzügler, ein Aufmerksamkeitsdefizit-/Hyperaktivitätsstörung behandelndes Mittel, ein kardiovaskuläres Mittel, ein Kalziumkanalblocker, ein Antianginosum, ein Zentralnervensystemmittel, ein Betablocker, ein Antiarrhythmikum, ein Bronchodilator, ein Zentralnervensystemstimulans, ein Diuretikum, ein genetisches Material, ein Hormonolytikum, ein Hypnotikum, ein hyperkalzämisches Mittel, ein hypoglykämisches Mittel, ein Immunsuppressivum, ein Antimuskarinikum, ein urogenitales Glattmuskelrelaxans, ein Betaagonist, ein narkotischer Antagonist, Nikotin, ein Ernährungsmittel, ein Parasympatholytikum, ein Peptid-Medikament, ein Antihämorrhoidikum, ein Psychostimulans, ein Psychotropikum, ein Mukolytikum, ein Sedativ, ein Laxativ, ein Vitamin, ein Sialagogum, ein Steroid, ein Sympathomimetikum, ein Beruhigungsmittel oder ein Vasodilator oder eine Kombination davon in Form von freier Base, freier Säure, pharmazeutisch verträglichem Salz, Solvat oder Hydrat davon ist.

4. Zusammensetzung nach Anspruch 2, wobei der Wirkstoff Dextromethorphan, Hydrocodon, Chlorpheniramin, Phenylephrin, Pseudoephedrin, Codein, Morphin, Dihydromorphon, Oxycodon, Dimenhydrinat, Diphenhydramin, Brompheniramin, Cetirizin, Levocetirizin, Ambroxol, Bromhexin, Carbocistein, Domiodol, Guaifenesin, Hydroxyzin, Dexbrompheniramin, Fexofenadin, Terfenadin, Dexchlorpheniramin oder Kombination davon in Form von freier Base, freier Säure, pharmazeutisch verträglichem Salz, Solvat oder Hydrat davon ist.

5. Zusammensetzung nach Anspruch 2, wobei das Ionenaustauschharz ein Kationenaustauschharz oder ein Anionenaustauschharz oder Kombination davon ist.

6. Zusammensetzung nach Anspruch 5, wobei das Kationenaustauschharz ein Copolymer von Methacrylsäure und Divinylbenzol, ein Natriumpolystyrolsulfonatharz, ein sulfoniertes Copolymer von Styrol und Divinylbenzol, ein vernetztes Polyacrylsäureharz, ein Polyacrylatharz, ein vernetztes Carboxylsäureharz, ein vernetztes Sulfonsäureharz, ein vernetztes Phosphonsäureharz, Zeolith oder eine Kombination davon ist.

7. Zusammensetzung nach Anspruch 2, wobei der Wirkstoff und das Ionenaustauschharz in einem Verhältnis von 1:0,1 bis 1:20 in dem Arzneimittel-Harz-Komplex vorliegen.

8. Zusammensetzung nach Anspruch 1, wobei die Freisetzungsmodifikator-Beschichtung mindestens einen Freisetzungsmodifikator umfasst, wobei der Freisetzungsmodifikator ein wasserunlöslicher Freisetzungsmodifikator, ein wasserlöslicher Freisetzungsmodifikator oder eine Kombination davon ist; wobei der wasserlösliche Freisetzungsmodifikator wahlweise aus Polyvinylpyrrolidon, Poloxamer, Guargummi, Xanthangumi, Gummi arabicum, Tragant, Cellulosederivaten wie Hydroxypropylmethylcellulose, Hydroxypropylcellulose, Methylcellulose und Hydroxyethylcellulose, Carboxymethylethylcellulose, Hydroxyethylmethylcarboxymethylcellulose, Hydroxyethylmethylcellulose, Carboxymethylcellulose, Methylhydroxyethylcellulose oder Methylhydroxypropylcellulose oder einer Kombination davon ausgewählt ist.

9. Zusammensetzung nach Anspruch 8, wobei der wasserunlösliche Freisetzungsmodifikator ein polymerer wasserunlöslicher Freisetzungsmodifikator, ein nicht polymerer wasserunlöslicher Freisetzungsmodifikator oder eine Kombination davon ist; wobei der polymere wasserunlösliche Freisetzungsmodifikator wahlweise aus Polyvinylacetat, Polyvinylchlorid, Polyvinylcarbonat, Ethylcellulose, Nitrocellulose, Vinylidenchlorid-Acrylonitril-Copolymer, Acrylonitril-Styrol-Copolymer, Ethylenvinylacetat, Celluloseacetat, Celluloseacetatphthalat, Celluloseacetatbutyrat, Copolymer von Vinylpyrrolidon, Hydroxypropylmethylcellulosephthalat, Methacrylsäure-Copolymer, oder Methacrylat-Copolymer oder einer Kombination davon ausgewählt ist.

10. Zusammensetzung nach Anspruch 1, wobei die Kügelchen mit verzögerter Freisetzung und die Zusammensetzung ferner mindestens einen pharmazeutisch verträglichen Exzipienten umfassen, wobei der pharmazeutisch verträgliche Exzipient aus Verdünnungsmittel, Stabilisator, Freisetzungsmodifikator, Bindemittel, Sprengmittel, Supersprengmittel, speichelflussförderndem Mittel, Tensid, Aroma, Süßstoff, Färbemittel, Säuerungsmittel, Viskosmacher, Gleitstoff, Schmiermittel, Lösungsvermittler, Stabilisator, Suspensionsmittel, Konservierungsmittel, Co-Lösungsmittel, Rieselhilfe oder Puffer oder einer Kombination davon ausgewählt ist.

11. Zusammensetzung nach Anspruch 1, wobei die Zusammensetzung mit verzögerter Freisetzung in Form einer flüssigen, einer festen oder einer halbfesten Zubereitung vorliegt; wobei es sich bei der flüssigen Zubereitung um Suspension handelt und es sich bei der festen Zubereitung um Kapsel, Tablette, Caplette, oral zerfallende Tablette, dispergierbare Tablette, Trockensuspension zur Rekonstitution, Granulat, Oblate oder Kautablette handelt.

12. Zusammensetzung nach Anspruch 1, wobei die Zusammensetzung mit verzögerter Freisetzung ferner einen zusätzlichen Wirkstoff umfasst, wobei der zusätzliche Wirkstoff wahlweise auf sofort freigesetzte oder verzögert freigesetzte Weise abgegeben wird.

## Revendications

1. Composition à libération prolongée comprenant une pluralité de billes à libération prolongée comprenant des complexes médicament-résine enrobés comprenant :
a. un complexe médicament-résine ; et
b. un revêtement modificateur de libération ;
lesdits complexes médicament-résine enrobés étant présents sous la forme d'au moins deux populations de complexes médicament-résine enrobés de manière variable comprenant au moins une population de complexes médicament-résine enrobés avec au moins un modificateur de libération représentant de 1 % à 15 % en poids du complexe médicament-résine ; et au moins une population de complexes médicament-résine enrobés avec au moins un modificateur de libération représentant de 15 % à 75 % en poids du complexe médicament-résine et au moins deux populations de complexes médicament-résine enrobés de manière variable étant présentes dans un rapport de 1:9 à 9:1 dans la quantité totale des complexes médicament-résine enrobés.

2. Composition selon la revendication 1, ledit complexe médicament-résine comprenant au moins un agent actif et au moins une résine échangeuse d'ions.

3. Composition selon la revendication 2, ledit agent actif étant un agent antitussif, un analgésique, un antihistaminique, un expectorant, un mucolytique, un décongestionnant, un agent analeptique, un agent anesthésique, un antiasthmatique, un agent antiarthritique ; un agent anticancer, un agent anticholinergique, un agent anticonvulsif, un agent antidépresseur, un antidiabétique, un agent antihelmintique, un agent antidiarrhéique ; un antiépileptique, un agent antihyperlipidémique ; un antihypertenseur, un antihypotenseur, un agent anti-infectieux, un agent antiinflammatoire, un agent antiinflammatoire non stéroïdien, un antiémétique, un agent antimigraineux ; un agent antinéoplasique, un agent antituberculeux, un antibiotique, un antiacide, un agent antiulcéreux ; un médicament antiparkinsonien, un agent antiprurigineux, un agent antipsychotique, un agent antipyrétique, un antispasmodique, un agent antiviral, un agent anxiolytique, un anorexigène, un agent de traitement du trouble d'hyperactivité avec déficit de l'attention, un agent cardiovasculaire, un inhibiteur des canaux calciques, un agent antiangineux, un agent du système nerveux central, un bêta-bloquant, un agent antiarythmique, un bronchodilatateur, un stimulant du système nerveux central, un diurétique, un matériel génétique, un hormonolytique, un hypnotique, un hypercalcémique, un agent hypoglycémique, un agent immunosuppresseur, un antimuscarinique, un relaxant des muscles lisses génito-urinaires, un bêta-agoniste, un antagoniste narcotique, la nicotine, un agent nutritionnel, un parasympatholytique, un médicament peptidique, un antihémorroïde, un psychostimulant, un psychotrope, un mucolytique, un sédatif, un laxatif, une vitamine, un sialagogue, un stéroïdien, un sympathomimétique, un tranquillisant ou un vasodilatateur ou une combinaison de ceux-ci sous la forme d'une base libre, d'un acide libre, d'un sel, solvate ou hydrate pharmaceutiquement acceptable de ceux-ci.

4. Composition selon la revendication 2, ledit agent actif étant le dextrométhorphane, l'hydrocodone, la chlorphéniramine, la phényléphrine, la pseudoéphédrine, la codéine, la morphine, la dihydromorphone, l'oxycodone, le dimenhydrinate, la diphenhydramine, la bromphéniramine, la cétirizine, la lévocétirizine, l'ambroxol, la bromhexine, la carbocistéine, le domiodol, la guaifénésine, l'hydroxyzine, la dexbromphéniramine, la fexofénadine, la terfénadine, la dexchlorphéniramine ou une combinaison de ceux-ci sous la forme d'une base libre, d'un acide libre, d'un sel, solvate ou hydrate pharmaceutiquement acceptable de ceux-ci.

5. Composition selon la revendication 2, ladite résine échangeuse d'ions étant une résine échangeuse de cations ou une résine échangeuse d'anions ou une combinaison de celles-ci.

6. Composition selon la revendication 5, ladite résine échangeuse de cations étant un copolymère d'acide méthacrylique et de divinylbenzène, une résine de sulfonate de polystyrène sodique, un copolymère sulfoné de styrène et de divinylbenzène, une résine d'acide polyacrylique réticulée, une résine de polyacrylate, une résine d'acide carboxylique réticulée, une résine d'acide sulfonique réticulée, une résine d'acide phosphonique réticulée, une zéolite ou une combinaison de ceux-ci.

7. Composition selon la revendication 2, ledit agent actif et ladite résine échangeuse d'ions étant présents dans un rapport de 1:0,1 à 1:20 dans le complexe médicament-résine.

8. Composition selon la revendication 1, ledit enrobage modificateur de libération comprenant au moins un modificateur de libération, ledit modificateur de libération étant un modificateur de libération insoluble dans l'eau, un modificateur de libération soluble dans l'eau ou une combinaison de ceux-ci ; ledit modificateur de libération soluble dans l'eau étant éventuellement choisi parmi la polyvinylpyrrolidone, le poloxamère, la gomme de guar, la gomme de xanthane, la gomme arabique, la gomme adragante, les dérivés de cellulose tels que l'hydroxypropylméthylcellulose, l'hydroxypropylcellulose, la méthylcellulose et l'hydroxyéthylcellulose, la carboxyméthyléthylcellulose, l'hydroxyéthylméthylcarboxyméthylcellulose, l'hydroxyéthylméthylcellulose, la carboxyméthylcellulose, la méthylhydroxyéthylcellulose ou la méthylhydroxypropylcellulose ou une combinaison de ceux-ci.

9. Composition selon la revendication 8, ledit modificateur de libération insoluble dans l'eau étant un modificateur de libération polymère insoluble dans l'eau, un modificateur de libération non polymère insoluble dans l'eau ou une combinaison de ceux-ci ; ledit modificateur de libération polymère insoluble dans l'eau étant éventuellement choisi parmi le polyacétate de vinyle, le polychlorure de vinyle, le polycarbonate de vinyle, l'éthylcellulose, la nitrocellulose, un copolymère chlorure de vinylidène-acrylonitrile, un copolymère acrylonitrile-styrène, l'éthylène-acétate de vinyle, l'acétate de cellulose, le phtalate d'acétate de cellulose, le butyrate d'acétate de cellulose, un copolymère de vinylpyrrolidone, le phtalate d'hydroxypropylmethylcellulose, un copolymère d'acide méthacrylique ou un copolymère de méthacrylate ou une combinaison de ceux-ci.

10. Composition selon la revendication 1, lesdites billes à libération prolongée et ladite composition comprenant en outre au moins un excipient pharmaceutiquement acceptable, ledit excipient pharmaceutiquement acceptable étant choisi parmi un diluant, un stabilisant, un modificateur de libération, un liant, un délitant, un super délitant, un agent de salivation, un tensioactif, un arôme, un édulcorant, un colorant, un agent d'aigreur, un agent viscolisant, un agent de glissement, un lubrifiant, un solubilisant, un stabilisant, un agent de mise en suspension, un conservateur, un co-solvant, un agent anti-agglomérant ou un tampon ou une combinaison de ceux-ci.

11. Composition selon la revendication 1, ladite composition à libération prolongée étant sous la forme d'un liquide, d'un solide ou d'une préparation semi-solide ; ladite préparation liquide étant une suspension et ladite préparation solide étant une capsule, un comprimé, un caplet, un comprimé à désintégration orale, un comprimé dispersible, une suspension sèche pour reconstitution, un granulé, une pastille ou un comprimé à dispersion rapide.

12. Composition selon la revendication 1, ladite composition à libération prolongée comprenant en outre un agent actif supplémentaire, ledit agent actif supplémentaire étant éventuellement administré selon un mode de libération immédiate ou de libération prolongée.
